# EUROPEAN PATENT SPECIFICATION

(11) **EP 2 168 559 B1**
(45) Date of publication and mention of the grant of the patent: **07.03.2012**
(21) Application number: 09170468.4
(22) Date of filing: 16.09.2009
(51) Int. Cl.: A61J 15/00, A61M 25/04

(54) **Skin level device for use with gastrostomy tube**
Hautvorrichtung zur Verwendung mit einem Gastrostomierohr
Dispositif au niveau cutané pour une utilisation avec un tube gastrostomique

(30) Priority: 30.09.2008 US 101231 P
(43) Date of publication of application: 31.03.2010
(73) Proprietor: Tyco Healthcare Group LP, Mansfield, MA 02048 (US)
(72) Inventor: Swisher, David Rork, St Charles, MO 63301 (US)
(74) Representative: Tomlinson, Edward James

(56) References cited:
- WO-A2-2006/133927
- WO-A2-2007/087254
- US-A- 3 915 171
- US-A1- 2002 177 806
- US-A1- 2009 182 264

## Description

### FIELD OF THE INVENTION

The present disclosure relates to gastrostomy tubes and, more particularly, to gastrostomy tubes including a skin level device having an expandable balloon and pad.

### BACKGROUND OF THE INVENTION

WO 2007/087254 A2 describes a catheter system for placement in a patient's bladder or into the stomach having internal and external retention bolsters. The appearance of the external bolster does not depend on the inflation state of the internal bolster.

Low profile gastrointestinal feeding systems are frequently used for long term tube fed patients who are mobile and/or combative and who require some type of gastrostomy device to provide nutrition to their stomachs. These gastrointestinal systems may include a feeding set having a nutrition source attached to one end and a low profile gastrostomy tube connected to the other end.

The low profile gastrostomy tube is normally inserted through an established, mature stoma extending through the patient's abdominal and stomach walls. The tube is held in place by an internal retention member (e.g., an inflatable balloon or other retention means, such as a plurality of flexible retaining arms) deployed inside a patient's stomach or other visceral organ to anchor the free end of the gastrostomy tube to the organ. The internal retention member affixes a hollow organ of choice, for example, the stomach against the posterior abdominal wall of a patient. To facilitate a proper fit, some low profile gastrostomy devices use a skin level device (e.g., a skin disk) that serves as a "gap filler" on the posterior abdominal wall of a patient. The skin level device is intended to provide a tight, wiggle free connection. US 2002/0177806 discloses such a tube

Typically, a daily cleaning regimen in and around the stoma site is performed to minimize odor and the risk of infection. Unfortunately, the configuration of the skin disk sometimes requires a clinician to work around the skin disk and, in some instances, even move or remove the skin disk during the cleaning regiment. This combination of working around the skin disk and/or removing the skin disk may cause discomfort to a patient and increase the risk that the skin disk will be reassembled improperly.

As noted, gastrostomy tubes may employ an inflatable balloon that anchors the free end of the gastrostomy tube to a patient's stomach. To inflate the balloon, a clinician typically inserts a tip of a syringe into a port or valve in communication with the gastrostomy tube to inflate the balloon with a predetermined volume of fluid contained in the syringe. A clinician typically cannot see the balloon while it is being inflated because the balloon is positioned in the patient's stomach. Thus, the clinician has no visual means of knowing whether or not the balloon has actually inflated to a predetermined volume. Under-inflation and over-inflation may cause the balloon not to function as intended. For example, balloon integrity may become compromised (e.g., the balloon may dry out and loose some of its elasticity). In this instance, the balloon may rupture, over expand, or under expand during inflation, which in turn, may prevent the balloon from properly anchoring a free end of the gastrostomy tube to a stomach wall. This may prove deleterious to the patient.

### SUMMARY

In one aspect of the invention, a gastrostomy tube comprises a body having a hub portion and a tubular portion extending distally from the hub portion. The hub portion includes a top surface and a bottom surface. The body defines a primary lumen having a first end in communication with an opening in the hub portion. The primary lumen extends through the body and has a second end in communication with an opening located in a distal end of the tubular portion. The gastrostomy tube also includes a port supported on the body. The port communicates with a secondary lumen. The port is adapted to engage an end of a luer tip syringe configured for injecting fluid into the gastrostomy tube. Further, the gastrostomy tube comprises an inflatable external balloon located at a proximal end of the body. The external balloon is positioned in use to engage an outer abdominal wall of a patient. The gastrostomy tube also comprises an inflatable internal balloon located distally from the external balloon and positioned on the tubular portion to engage an interior wall of a stomach of the patient in use. The internal and external balloons are in fluid communication with the port via the secondary lumen such that each of the external and internal balloons is inflatable from a deflated condition to an inflated condition. The internal and external balloons are substantially identical to each other and configured to enable a clinician to indirectly observe the internal balloon by observing the external balloon.

In another aspect, not part of the invention, a gastrostomy tube comprises a body having a hub portion and a tubular portion extending distally from the hub portion. The hub portion includes a top surface and a bottom surface. The body defines a primary lumen having a first end in communication with an opening in the hub portion. The primary lumen extends through the body and has a second end in communication with an opening located in a distal end of the tubular portion. The gastrostomy tube further comprises a plurality of valves supported on the body. Each of the plurality of valves communicates with a respective secondary lumen and is adapted to engage an end of a luer tip syringe configured for injecting fluid into the gastrostomy tube. In addition, the gastrostomy tube includes an inflatable external balloon located at a proximal end of the body. The external balloon is positioned in use to engage an outer abdominal wall of a patient. Further, the gastrostomy tube comprises an inflatable internal balloon located distally from the external balloon and positioned on the tubular portion to engage an interior wall of a stomach of the patient in use. Each of the internal and external balloons is in fluid communication with at least one of the plurality of valves via one of their respective secondary lumens such that each of the external and internal balloons is inflatable from a deflated condition to an inflated condition. The external balloon can be deflated while the internal balloon is inflated to facilitate cleaning of an area adjacent a stoma site of the patient.

In yet another aspect, not part of the invention, a gastrostomy tube comprises a body having a hub portion and a tubular portion extending distally from the hub portion. The hub portion includes a top surface and a bottom surface. The body defines a primary lumen having a first end in communication with an opening in the hub portion. The primary lumen extends through the body and has a second end in communication with an opening located in a distal end of the tubular portion. The gastrostomy tube also includes a port supported on the body. The port communicates with a secondary lumen. Further, the gastrostomy tube comprises an expandable inflatable external inflatable balloon located at a proximal end of the body. The external balloon is positioned in use to engage an outer abdominal wall of a patient. The external balloon is in fluid communication with the port via the secondary lumen such that the external balloon is inflatable from a deflated condition to an inflated condition via the port. The gastrostomy tube also comprises an internal securement device located distally from the external balloon and positioned on the tubular portion to engage an interior wall of a stomach of the patient in use. The external balloon can be deflated in use while the internal securement device is in an expanded condition to facilitate cleaning of an area adjacent a stoma site of the patient.

### BRIEF DESCRIPTION OF THE DRAWINGS

Various embodiments of the presently disclosed low profile gastrostomy tube are disclosed herein with reference to the drawings wherein:

Fig.1 is a perspective illustrating a low profile gastrostomy tube including a pair of inflatable balloons in an inflated state in accordance with a first embodiment of the present disclosure;

Fig. 2A is a side view illustrating the low profile gastrostomy tube of the first embodiment engaging an abdominal wall and a stomach wall;

Fig. 2B is a side view illustrating a low profile gastrostomy tube engaging an abdominal wall and a stomach wall in accordance with a second embodiment of the present disclosure;

Fig.3A is a side view illustrating a low profile gastrostomy tube engaging an abdominal and stomach wall in accordance with an example;

Fig. 3B is a perspective illustrating a low profile gastrostomy tube having a pair of inflatable balloons in an inflated state in accordance with an example;

Fig. 3C is a perspective illustrating a low profile gastrostomy tube having an inflatable balloon in an inflated state and an internal securement device having a plurality of flexible retaining arms in accordance with an example.

### DETAILED DESCRIPTION OF EMBODIMENTS

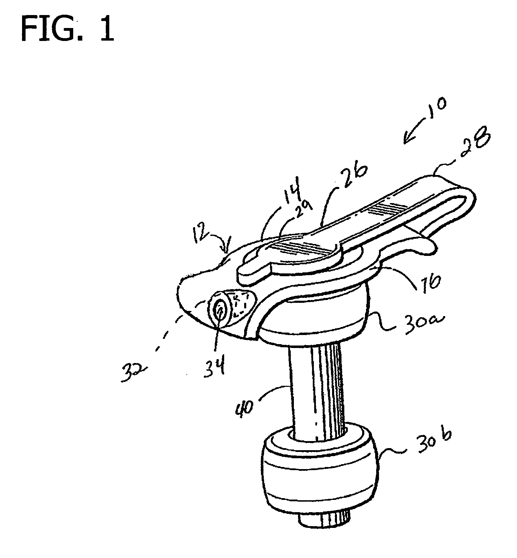

Referring to the drawings, a first embodiment of a low profile gastrostomy tube according to the present disclosure is illustrated and generally indicated as 10 in Fig. 1. The gastrostomy tube 10 may be configured for use with low profile gastrointestinal feeding systems. Known low profile gastrointestinal feeding systems suitable for use with the gastrostomy tube 10 of the present disclosure typically include a feeding set (not shown) having an elongate tube attachable to a fluid source and a connection member for securing the elongate tube to, for example, a low profile gastrostomy tube 10. A more detailed description of a feeding set suitable for use with the gastrostomy tube 10 of the present disclosure is provided in commonly owned U.S. Patent Nos. 7,070,587 and 6,045,536 both to Meier et al., both of which are incorporated herein by reference in their entirety.

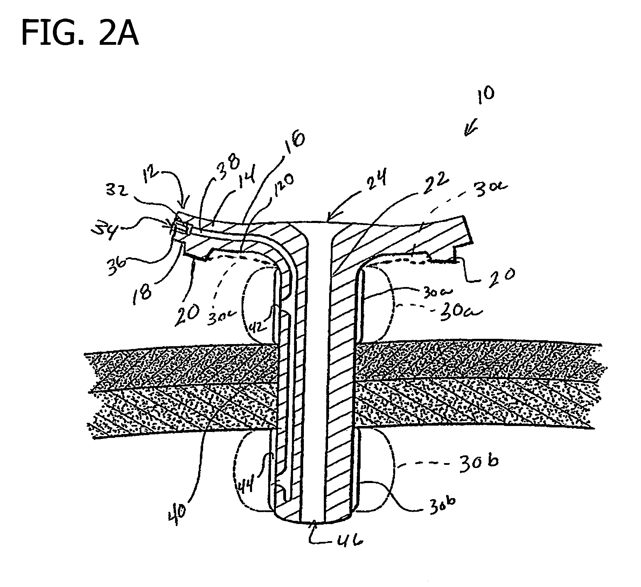

Referring to Figs. 1 and 2A, the gastrostomy tube 10 includes a body 12 having a hub portion 14 (hub 14) and a tubular portion 40 extending distally from the hub 14. The hub 14 includes a top surface 16 and a bottom surface 18 (Fig. 2A). In some embodiments, the bottom surface 18 forms opposing legs 20 (Fig.2A) configured and dimensioned to seat against an outer abdominal wall of a patient when a skin level device, e.g., an external inflatable balloon 30a, is in its deflated state (e.g., when cleaning a stoma). Alternatively, the bottom surface 18 of the body 12 may be substantially flat and not include legs 20. In the drawings, the size of opposing legs 20 has been exaggerated for illustrative purposes.

The gastrostomy tube 10 includes a primary lumen 22 extending through the body 12 in communication with a proximal opening 24. As shown in Fig. 1, a tethered cap 26 is affixed to or integrally formed with the hub 14 (Fig. 1). The cap 26 includes a tether 28 attaching a cap member 29 to the hub 14 (Fig. 1). The cap member 29 may be configured to seal the primary lumen 22. An annular undercut may be formed around the circumference of the primary lumen 22 near the opening 24 of the lumen for engaging an annular flange (not shown) of the cap member 29 to secure the cap member in the primary lumen 22. Engagement between the annular flange and the undercut is preferably a snap fit engagement. Alternatively, an interference fit or a combination of interference and snap fit engagement may be used for the same purpose. The hub 14 may also include a valve member (not shown) positioned across the primary lumen 22 for sealing off the primary lumen to fluid flow. For a more detailed description of the aforementioned components, parts, and/or members associated with the low profile gastrostomy tube 10, reference may be made to commonly owned U.S. Patent No. 7,070,587.

The hub 14 includes one or more ports having one or more valves 32 (hereinafter collectively referred to as valves 32 and shown in phantom in Fig. 1 and schematically in Fig. 2A) extending longitudinally from the body 12. The valve 32 includes an opening 34 that communicates with a passage 36 (Fig. 2A) and is adapted to engage an end of a luer tip syringe (not shown) or other suitable device for injecting fluid into the gastrostomy tube 10 to inflate one or more of the inflatable balloons as shall be discussed in greater detail below. The valve member 32 is positioned across the passage 36 for providing a fluid tight barrier allowing fluid to enter the valve when the luer tip syringe is properly engaged to it. However, when the end of the luer tip syringe is disengaged from the valve 32, the valve portion reseals itself, preventing fluid from escaping from the valve. Such valves are known in the art and will not be described in further detail.

With reference to Fig. 2A, the passage 36 communicates with a secondary lumen 38 extending through the hub 14 and communicating with an external balloon 30a and an internal balloon 30b through a tubular portion 40 of the gastrostomy tube 10. The secondary lumen 38 extends axially through the tubular portion 40 and terminates within interiors 42, 44 of the balloons 30a, 30b, respectively, attached to or integral with the tubular portion 40.

As illustrated in Fig. 2A, both the primary lumen 22 and the secondary lumen 38 extend axially through the tubular portion 40 with the primary lumen terminating at a distal opening 46 and the secondary lumen communicating with the interiors 42, 44 of the inflatable balloons 30a, 30b, respectively. The secondary lumen 38 provides a fluid conduit between the valve 32 and the inflatable balloons 30a, 30b to effect inflation/deflation of the balloons and to facilitate securing and removing the gastrostomy tube 10 with respect to the stoma site.

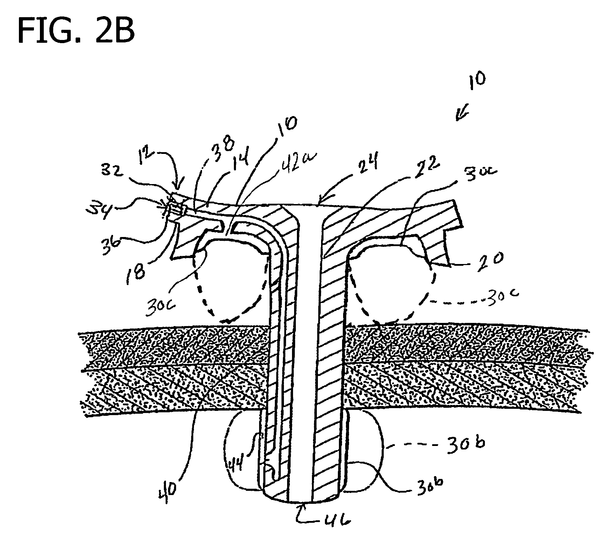

The external inflatable balloon 30a is configured for seating the gastrostomy tube 10 on the outer abdominal wall of a patient and acting as a "gap filler" between the bottom surface 18 of the hub 14 and the outer abdominal wall of a patient to obtain a tight, wiggle free connection between the gastrostomy tube and the patient. In the drawings, the height of the low profile gastrostomy tube 10 relative to an outer abdominal wall of a patient has been exaggerated for illustrative purposes. The external balloon 30a may also act as a secondary seal at the stoma site. With this purpose in mind, the external inflatable balloon 30a is located on the proximal end of the tubular member 40 adjacent the hub 14. Alternatively, instead of the balloon 30a, an external balloon 30c may be operatively disposed on the bottom surface 18 of the hub 14 (as shown in phantom in Fig. 2A). In this instance, the secondary lumen 38 would be configured to communicate with an interior 42a of the balloon 30c so the balloon 30c could be expanded to perform the above-described functions of the balloon 30a (see Fig. 2B, for example).

The internal inflatable balloon 30b is configured for affixing a hollow organ, i.e., the stomach, against the posterior abdominal wall of the patient. Each of the inflatable balloons 30a, 30b, or 30c is constructed from an elastomeric or other flexible material of similar thickness permitting the inflatable balloons 30a, 30b to assume an inflated state when fluid is injected into their respective interiors 42, 44 through the secondary lumen 38.

In use, the gastrostomy tube 10 is inserted through a stoma on a patient (Fig. 2A) while the balloons 30a, 30b are in a deflated state. To inflate the balloons 30a, 30b, a clinician engages the tip of a syringe to the valve 32 and injects fluid into the passage 36 and through the secondary lumen 38. As fluid travels through the secondary lumen 38 it enters the interiors 42, 44 of the balloons 30a, 30b (or 30c) and inflates the respective balloons 30a, 30b (shown in phantom in Figs. 2A and 2B, for example). As noted above, the height of the low profile gastrostomy tube 10 relative to an outer abdominal wall of a patient when the external balloons 30a or 30c are inflated has been exaggerated for illustrative purposes. Once the balloons 30a, 30b are inflated, the gastrostomy tube 10 is intended to remain comfortably and securely connected to a patient so the primary lumen 22 may act as a conduit for delivering fluid directly into the patient's stomach or other visceral organ. To deflate the balloons 30a, 30b (e.g., to clean in and/or around the stoma), the clinician withdraws fluid from the interiors 42, 44 and through the valve 32 by pulling back on the plunger of a syringe until all the fluid has been fully evacuated from the balloons 30a, 30b. Because the internal balloon 30a and the external balloon 30b are substantially identical and formed from the same material, have the same thicknesses and are interconnected by a single lumen 38, the volume of the internal balloon 30b will correspond to the volume of the external balloon 30a. Thus, the clinician may observe the external balloon 30a to obtain an estimate of the size of the internal balloon 3b. This configuration aides the clinician in accurately inflating the internal balloon 30b.

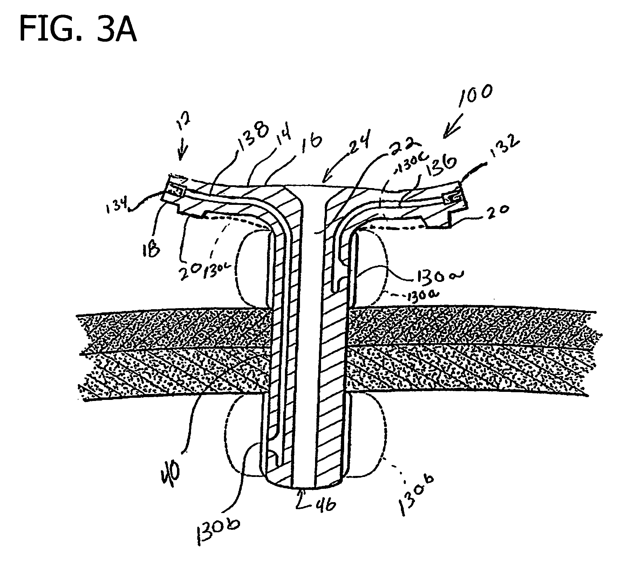

Referring now to Fig. 3A, an example of a low profile gastrostomy tube is illustrated and generally indicated as 100. In the example illustrated in Fig 3A, a pair of valves 132, 134 (shown schematically) are positioned on opposite sides of the hub 14. The operative features of the gastrostomy tube 100 are substantially similar to the gastrostomy tube 10. However, in the example illustrated in Fig. 3A, the gastrostomy tube 100 includes two valves 132, 134 that independently communicate with the balloons 130a (or 130c) and 130b, respectively, by way of respective secondary lumens 136, 138. In this instance, each of the balloons 130a (or 130c) and 130b is independently inflatable and deflatable by way of their respective valves 132, 134. This combination of valves 132, 134 and balloons 130a (or 130c) and 130b facilitates cleaning in and around the stoma site, because the external balloon can be deflated without deflating the internal balloon. Deflating the external balloon 130a allows for greater access to the stoma site to enable a clinician to clean around the stoma site while maintaining the gastrostomy tube 100 secured to a patient.

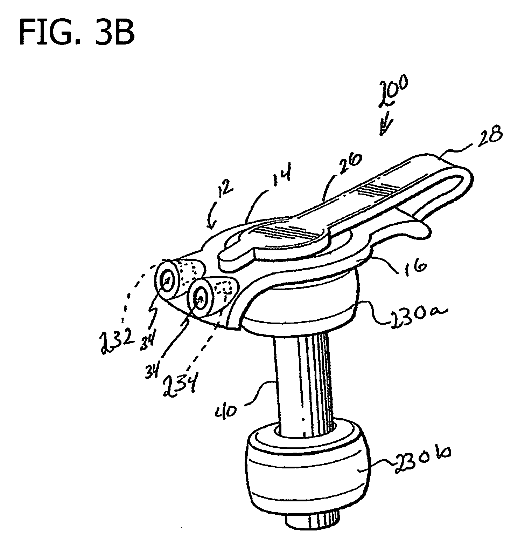

Referring now to Fig. 3B, another example of a low profile gastrostomy tube according to the present disclosure is illustrated and generally indicated as 200. ln the example illustrated in Fig. 3B, the gastrostomy tube 200 is shown having two valves 232, 234 (shown in phantom) configured to independently communicate with the balloons 230a, 230b. In this instance, the valves 232, 234 are positioned on the same side of the hub 14 and adjacent one another. The operative features of gastrostomy tube 200 are substantially similar to gastrostomy tube 100. As with each of the balloons 130a, 130b described above with respect to the previous embodiment, each of the balloons 230a, 230b of this embodiment are independently inflatable and deflatable by way of their respective valves 232, 234.

While the above-described gastrostomy tubes (e.g., gastrostomy tube 10) of the present disclosure have been described in terms of use with an internal balloon member (e.g., 30b) as a means for securing the gastrostomy tube inside a visceral organ of a patient, other means for securing the tube are contemplated.

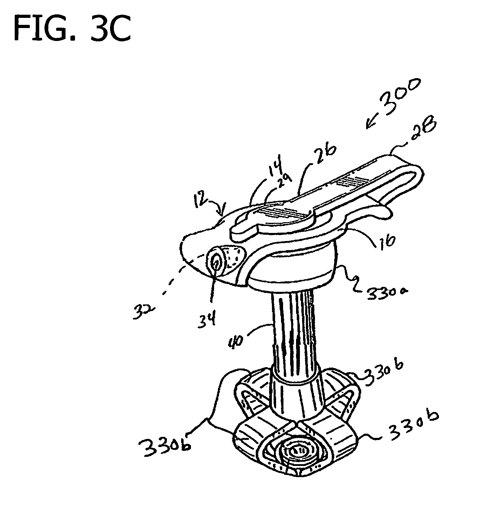

For example, referring now to Fig. 3C, yet another example of a low profile gastrostomy tube is illustrated and generally indicated as 300. In the example illustrated in Fig. 3C, the gastrostomy tube 300 is shown having an internal securement device including a cage having one or more flexible arms 330b (four flexible arms are shown). The flexible arms 330b are movable from a collapsed configuration enabling a clinician to insert the gastrostomy tube 300 through an established, mature stoma of a patient to a expanded configuration (Fig. 3C) enabling a clinician to anchor or secure the stomach against the posterior abdominal wall of a patient. For a more detailed description of the operative features of the flexible arms 330b reference can be again made to commonly owned U.S. Patent No. 7,070,587. In the embodiment illustrated in Fig. 3C, a secondary lumen (see Fig. 2A for example) would be configured to communicate with an interior 42 of the balloon 330a so the balloon can be expanded to perform the above-described functions of the balloon 30a.

It will be understood that various modifications may be made to the embodiments disclosed herein. Therefore, the above description should not be construed as limiting, but merely as examples of preferred embodiments.

## Claims

1. A gastrostomy tube (10) comprising:
a body (12) having a hub portion (14) and a tubular portion (40) extending distally from the hub portion, the hub portion including a top surface and a bottom surface, the body defining a primary lumen (22) having a first end in communication with an opening in the hub portion, the primary lumen extending through the body and having a second end in communication with an opening located in a distal end of the tubular portion;
a port (32) supported on the body, the port communicating with a secondary lumen (38), the port being adapted to engage an end of a luer tip syringe configured for injecting fluid into gastrostomy tube: and an inflatable internal ballon (30b)
**characterized in that** it further comprises
an inflatable external ballon (30a) located at a proximal end of the body, the external balloon being positioned in use to engage an outer abdominal wall of a patient; and
said inflatable internal balloon (30b) located distally from the external balloon and positioned on the tubular portion to engage an interior wall of a stomach of the patient in use, the internal and external balloons being in fluid communication with the port via the secondary lumen such that each of the external and internal balloons is inflatable from a deflated condition to an inflated condition;
wherein the internal and external balloons are substantially identical to each other and configured to enable a clinician to indirectly observe the internal balloon by observing the external balloon and the external balloon is operatively disposed on the bottom surface of the hub portion.

2. The gastrostomy tube according to claim 1, wherein the gastrostomy tube is configured for use with a low profile gastrointestinal feeding system.

3. The gastrostomy tube according to claim 1, wherein the hub portion includes a bottom surface (18) forming opposing legs (20) configured and dimensioned to seat against the outer abdominal wall of a patient in use.

4. The gastrostomy tube according to claim 1, wherein the external balloon is configured to provide a secondary seal between the outer abdominal wall and an interior wall of a stomach of a patient in use.

5. The gastrostomy tube according to claim 1, wherein the external balloon is in fluid communication with a valve via the secondary lumen such that each of the external and internal balloons is inflatable from a deflated condition to an inflated condition.

6. The gastrostomy tube according to claim 1, wherein the hub portion includes a tethered cap (26) which includes a tether (28) attaching a cap member (29) to the opening of the hub portion.

## Patentansprüche

1. Gastrostomie-Tubus (10), der Folgendes umfasst:
einen Körper (12) mit einem Ansatzteil (14)und einem röhrenförmigen Abschnitt (40), der sich distal vom Ansatzteil erstreckt, wobei der Ansatzteil eine Oberseite und eine Unterseite aufweist und der Körper ein primäres Lumen (22) mit einem ersten mit einer Öffnung im Ansatzteil in Verbindung stehenden Ende definiert, wobei sich das primäre Lumen durch den Körper erstreckt und ein zweites Ende aufweist, das mit einer in einem distalen Ende des röhrenförmigen Abschnitts angeordneten Öffnung in Verbindung steht;
einen Port (32), der auf dem Körper gestützt wird und mit einem sekundären Lumen (38) in Verbindung steht, wobei der Port in ein Ende einer Luer-Spritze zur Injektion von Flüssigkeit in den Gastrostomie-Tubus eingreifen kann; und
einen aufblasbaren Innenballon (30b), **dadurch gekennzeichnet, dass** er ferner einen aufblasbaren Außenballon (30a) umfasst, der an einem proximalen Ende des Körpers angeordnet ist, wobei der Außenballon im Gebrauch so angeordnet ist, dass er in eine äußere Bauchwand eines Patienten eingreift; und wobei der aufblase Innenballon (30b) distal vom Außenballon angeordnet ist und so auf dem röhrenförmigen Abschnitt angeordnet ist, dass er in eine Innenwand eines Magens des Patienten im Gebrauch eingreift, wobei der Innenballon und der Außenballon über das sekundäre Lumen mit dem Port in Verbindung stehen, so dass der Außen- und Innenballon jeweils von einem entleerten Zustand in einen aufgeblasenen Zustand aufgeblasen werden kann; worin der Innenballon und der Außenballon im Wesentlichen identisch sind und so konfiguriert sind, dass sie einem Arzt die indirekte Beobachtung des Innenballons durch Beobachtung des Außenballons ermöglichen und der Außenballon wirksam auf der Unterseite des Ansatzteils angeordnet ist.

2. Gastrostomie-Tubus nach Anspruch 1, worin der Gastrostomie-Tubus zum Gebrauch mit einem gastroinstestinalen Niederprofil-Zufuhrsystem konfiguriert ist.

3. Gastrostomie-Tubus nach Anspruch 1, worin der Ansatzteil eine Unterseite (18) aufweist, die gegenüberliegende Schenkel (20) bildet, die so konfiguriert sind und solche Abmessungen aufweisen, dass sie an der äußeren Bauchwand eines Patienten im Gebrauch sitzen.

4. Gastrostomie-Tubus nach Anspruch 1, worin der Außenballon eine sekundäre Dichtung zwischen der äußeren Bauchwand und einer Innenwand eines Magens eines Patienten im Gebrauch bereitstellen kann.

5. Gastrostomie-Tubus nach Anspruch 1, worin der Außenballon über ein sekundäres Lumen mit einem Ventil in Flüssigkeitsverbindung steht, so dass der Außenballon und der Innenballon jeweils von einem entleerten Zustand in einen aufgeblasenen Zustand aufgeblasen werden können.

6. Gastrostomie-Tubus nach Anspruch 1, worin der Ansatzteil eine mit Zugseilen versehene Kappe (26) aufweist, die ein Zugseil (28) aufweist, das ein Kappenelement (29) an der Öffnung des Ansatzteils befestigt.

## Revendications

1. Tube (10) de gastrostomie comportant :
un corps (12) présentant une partie (14) embout et une partie (40) tubulaire s'étendant distalement depuis la partie embout, la partie embout comprenant une surface supérieure et une surface inférieure, le corps délimitant un canal (22) primaire possédant une première extrémité communiquant avec une ouverture dans la partie embout, le canal primaire traversant le corps et possédant une seconde extrémité communiquant avec une ouverture située dans une extrémité distale de la partie tubulaire ;
un orifice (32) supporté sur le corps, l'orifice communicant avec un canal (38) secondaire, l'orifice étant conçu pour mettre en prise une extrémité d'une seringue à pointe Luer configurée pour injecter un fluide dans le tube de gastrostomie ; et un ballonnet (30b) interne gonflable
**caractérisé en ce qu'**il comporte en outre un ballonnet (30a) externe gonflable situé au niveau d'une extrémité proximale du corps, le ballonnet externe étant positionné lors de l'utilisation de façon à mettre en prise une paroi abdominale externe d'un patient ; et
ledit ballonnet (30b) interne gonflable étant situé distalement par rapport au ballonnet externe et positionné sur la partie tubulaire pour mettre en prise une paroi intérieure de l'estomac du patient lors de l'utilisation, les ballonnets interne et externe étant en communication fluidique avec l'orifice par l'intermédiaire du canal secondaire de sorte que chacun des ballonnets externe et interne est gonflable depuis un état dégonflé jusqu'à un état gonflé ;
dans lequel les ballonnets interne et externe sont sensiblement identiques l'un par rapport à l'autre et configurés pour permettre à un clinicien d'observer indirectement le ballonnet interne en observant le ballonnet externe et le ballonnet externe est disposé de manière fonctionnelle sur la surface inférieure de la partie embout.

2. Tube de gastrostomie selon la revendication 1, dans lequel le tube de gastrostomie est configuré pour être utilisé avec un système d'alimentation gastrointestinal compact.

3. Tube de gastrostomie selon la revendication 1, dans lequel la partie embout comprend une surface (18) inférieure formant des branches (20) opposées configurées et dimensionnées pour s'appuyer contre la paroi abdominale externe d'un patient lors de l'utilisation.

4. Tube de gastrostomie selon la revendication 1, dans lequel le ballonnet externe est configuré pour fournir un joint d'étanchéité secondaire entre la paroi abdominale externe et une paroi intérieure de l'estomac d'un patient lors de l'utilisation.

5. Tube de gastrostomie selon la revendication 1, dans lequel le ballonnet externe est en communication fluidique avec une valve par l'intermédiaire du canal secondaire de sorte que chacun des ballonnets externe et interne est gonflable depuis un état dégonflé jusqu'à un état gonflé.

6. Tube de gastrostomie selon la revendication 1, dans lequel la partie embout comprend une coiffe (26) retenue qui comprend un dispositif de retenue (28) attachant un élément (29) de coiffe à l'ouverture de la partie embout.
